(19) Europäisches Patentamt
European Patent Office
Office européen des brevets

(11) EP 1 572 697 B1

(12) EUROPÄISCHE PATENTSCHRIFT

(45) Veröffentlichungstag und Bekanntmachung des Hinweises auf die Patenterteilung:
**21.02.2007 Patentblatt 2007/08**

(21) Anmeldenummer: **03813104.1**

(22) Anmeldetag: **01.12.2003**

(51) Int Cl.:
***C07D 493/04*** $^{(2006.01)}$ ***C12N 1/00*** $^{(2006.01)}$

(86) Internationale Anmeldenummer:
**PCT/EP2003/013484**

(87) Internationale Veröffentlichungsnummer:
**WO 2004/055021 (01.07.2004 Gazette 2004/27)**

(54) **SPIROBENZOFURANLACTAM-DERIVATE, VERFAHREN ZU IHRER HERSTELLUNG UND VERWENDUNG DERSELBEN**

SPIROBENZOFURANLACTAM DERIVATIVES, METHODS FOR THEIR PREPARATION, AND USE THEREOF

DERIVES DE SPIROBENZOFURANELACTAME, LEURS PROCEDES DE PREPARATION ET LEUR UTILISATION

(84) Benannte Vertragsstaaten:
**AT BE BG CH CY CZ DE DK EE ES FI FR GB GR HU IE IT LI LU MC NL PT RO SE SI SK TR**

(30) Priorität: **13.12.2002 DE 10258650**

(43) Veröffentlichungstag der Anmeldung:
**14.09.2005 Patentblatt 2005/37**

(73) Patentinhaber: **Sanofi-Aventis Deutschland GmbH**
**65929 Frankfurt am Main (DE)**

(72) Erfinder:
- **EDER, Claudia**
  **65719 Hofheim (DE)**
- **KURZ, Michael**
  **65719 Hofheim (DE)**
- **TOTI, Luigi**
  **65239 Hochheim (DE)**

(56) Entgegenhaltungen:
**WO-A-92/10096** **GB-A- 1 420 528**

- **ROGGO B E ET AL: "Novel Spirohydrobenzofuranlactams as Antagonists of Endothelin and as Inhibitors of HIV-1 Protease Produced by Stachybotrys sp" JOURNAL OF ANTIBIOTICS, JAPAN ANTIBIOTICS RESEARCH ASSOCIATION. TOKYO, JP, Bd. 49, Nr. 4, April 1996 (1996-04), Seiten 374-379, XP002146637 ISSN: 0021-8820**
- **R.M. EPPLEY ET AL: "Structures of Satratoxin F and Satatroxin G, metabolites of Stachybotrys atra" JOURNAL OF ORGANIC CHEMISTRY., Bd. 45, 1980, Seiten 2522-2523, XP002279123 EASTON US**
- **DENG ET AL: "Total synthesis and structure revision of Stachybotrys spirolactams" JOURNAL OF ORGANIC CHEMISTRY., Bd. 68, 2003, Seiten 7422-7427, XP002279124 EASTON US**
- **KENDE ET AL: "Enantioselective total synthesis and structure revision of spirobenzofuranlactam. Total synthesis of Stachybotrylactam" ORGANIC LETTERS, Bd. 5, Nr. 10, 2003, Seiten 1785-1788, XP002279125**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Wirkstoffe (Spirobenzofuranlactam-Derivate), die von dem Mikroorganismus Stachybotris atra ST002348, DSM 14952, während der Fermentation gebildet werden, Verfahren zu deren Herstellung, deren Verwendung als Arzneimittel, Spirobenzofuranlactam-Derivate enthaltende Arzneimittel sowie den Mikroorganismus Stachybotris atra ST002348, DSM 14952.

Das Plasminogen-Aktivierungssystem umfaßt eine Enzymkaskade, welche die kontrollierte, lokale Bildung des proteolytischen Enzyms Plasmin ermöglicht. Die Bildung von Plasmin spielt eine wichtige Rolle bei einer Vielzahl von physiologischen und pathophysiologischen Prozessen. Innerhalb dieser Enzymkaskade wird die Umwandlung des Zymogens Plasminogen in das proteolytisch aktive Plasmin entweder durch tPA (tissue type plasminogen activator) oder uPA (urokinase type plasminogen activator) aktiviert. Die Plasminaktivtät kann auf verschiedenen Ebenen kontrolliert bzw. geregelt werden. Die Aktivität von tPA und uPA wiederum wird durch PAI-1 und PAI-2 kontrolliert. Während tPA der wichtigste Plasminogen-Aktivator bei der Fibrinolyse ist, spielt uPA eine wichtige Rolle bei der Plasminbildung an Stellen, wo ein Abbau extrazellulärer Matrix stattfindet. Während uPA sowohl durch PAI-1 (Plasminogen-Aktivator Inhibitor 1) als auch PAI-2 reguliert wird, gibt es Hinweise darauf, daß tPA nur von PAI-1 beeinflusst wird. PAI-1 spielt somit eine wichtige Rolle bei der Aufrechterhaltung des Gleichgewichts zwischen Fibrinbildung und Fibrinolyse (J.D. Vassalli et al., J. Clin. Invest., 1991, 88, 1067-1072). Viele Studien erbrachten Hinweise darauf, daß ein erhöhter PAI-1 Spiegel einen Risikofaktor für kardiovaskuläre Erkrankungen darstellt. Erhöhte PAI-1 Konzentrationen wurden unter anderem nachgewiesen bei koronarer Herzerkrankung, akutem Myokardinfarkt, instabiler Angina pectoris, Venenthrombose und venösen Thromboembolien (P.J. Declerck et al., J. Intern. Med., 1994, 236, 425-432; H.A. Dawsons, Atherosclerosis, 1992, 95, 105-117). Diese klinischen Studien weisen darauf hin, daß PAI-1 ein neuartiges Target darstellt für die Behandlung von Erkrankungen, die mit einer verminderten Fibrinolyse einhergehen, beispielsweise verminderter Wundheilung (Charlton P., Exp. Opin. Invest. Drugs, 1997, 6, 539-554).

Erhöhte PAI-1 Werte werden auch mit arterieller Thrombose, Arteriosklerose, Insulinresistenz und makrovaskulären Verletzungen bei Typ-II Diabetes mellitus-Patienten, Hypoxie, septischem Schock, Lungenentzündung und Lungenfibrose in Verbindung gebracht, sowie darüber hinaus mit Krebserkrankungen, insbesondere Brustkrebs, Darmkrebs, Magenkrebs, Leberkrebs, Gehirntumoren, Ovarialtumoren, Speiseröhrenkrebs, Nierenkrebs, Muskelzellkarzinom, insbesondere Kopf- und Nackenmuskelkarzinom, wobei PAI-1 eine Schlüsselrolle beim Fortschreiten und der Metastasierung von Krebserkrankungen zugesprochen wird, insbesondere bei der Proteolyse, Adhäsion, Mobilisierung, Invasion, Chemotaxe, Proliferation und Angiogenese (P. Carlton, Exp. Opin. Invest. Drugs (1997), 6(5), 539-554; F. Frankenne et al., Expert Opinion on Therapeutic Targets, 1999, 3(3), 469-481). Eine Behandlung der einleitend genannten Erkrankungen ist daher durch Inhibierung von PAI-1 möglich.

Aufgabe der vorliegenden Erfindung ist es daher, Inhibitoren des Plasminogen-Aktivator Inhibitor 1 (PAI-1) bereitzustellen.

Es ist überraschend gefunden worden, dass der Mikroorganismus-Stamm Stachybotris atra ST002348, DSM 14952, Verbindungen zu bilden vermag, die den Plasminogen-Aktivator Inhibitor 1 (PAI-1) in sehr geringen Konzentrationen wirksam hemmen. Die Verbindungen der Formel hemmen die Inhibierung der enzymatischen Aktivität von tPA durch PAI-1 und sind demzufolge geeignet für die Behandlung und/oder Prophylaxe von koronarer Herzerkrankung, akutem Myokardinfarkt, instabiler Angina pectoris, Venenthrombose und venösen Thromboembolien, arterieller Thrombose, Arteriosklerose, Insulinresistenz und makrovaskulären Verletzungen bei Typ-II Diabetes mellitus-Patienten, Hypoxie, septischem Schock, Lungenentzündung und Lungenfibrose, sowie Krebserkrankungen, insbesondere Brustkrebs, Darmkrebs, Magenkrebs, Leberkrebs, Gehirntumoren, Ovarialtumoren, Speiseröhrenkrebs, Nierenkrebs, Muskelzellkarzinom, insbesondere Kopf- und Nackenmuskelkarzinom. Insbesondere sind die erfindungsgemäßen Verbindungen für eine antithrombotische Therapie zur Behandlung und Prophylaxe bei Patienten mit koronaren Herzerkrankungen und thrombotischen Erkrankungen des peripheren, venösen Systems geeignet.

Die Erfindung betrifft somit Verbindungen der Formel (I), im folgenden auch Spirobenzofuranlactam-Derivate genannt,

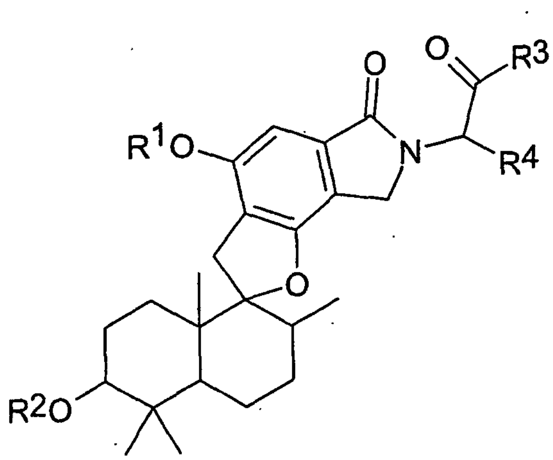

(I)

wobei

$R^1$ und $R^2$ unabhängig voneinander H, $C_1$-$C_6$-Alkyl, $C_2$-$C_6$-Alkenyl, $C_2$-$C_6$-Alkinyl oder $C_5$-$C_{14}$-Aryl bedeutet, worin Alkyl, Alkenyl, Alkinyl und Aryl unsubstituiert oder ein- bis dreifach durch einen Rest aus der Gruppe -OH, =O, -O-$C_1$-$C_6$-Alkyl, -O-$C_2$-$C_6$-Alkenyl, -O-$C_5$-$C_{14}$-Aryl, -NH-$C_1$-$C_6$-Alkyl, -NH-$C_2$-$C_6$-Alkenyl, -NH[-C(=O)-($C_1$-$C_6$-Alkyl)], -NH[-C(=O)-($C_5$-$C_{14}$-Aryl)], -$NH_2$ oder Halogen substituiert sind,

$R^3$ -OH, -O-$R^1$ oder-NH-$R^1$, und

$R^4$ H, $C_1$-$C_6$-Alkyl, $C_2$-$C_6$-Alkenyl, $C_5$-$C_{14}$-Aryl oder-($C_1$-$C_6$-Alkyl)-($C_5$-$C_{14}$-Aryl) bedeutet,

sowie deren physiologisch verträglichen Salze und/oder offensichtliche chemische Äquivalente.

Vorzugsweise sind $R^1$ und $R^2$ unabhängig voneinander gleich H oder $C_1$-$C_6$-Alkyl, besonders bevorzugt H, $R^3$ gleich OH oder -O-($C_1$-$C_6$-Alkyl), besonders bevorzugt OH, und $R^4$ gleich $C_1$-$C_6$-Alkyl oder -($C_1$-$C_6$-Alkyl)-($C_5$-$C_{14}$-Aryl), besonders bevorzugt Benzyl, 2-Butyl oder 1-(2-Methyl-propyl).

Besonders bevorzugt ist eine Verbindung der Formel (I), wobei $R^1$ und $R^2$ H bedeuten, $R^3$ OH bedeutet und $R^4$ die oben genannte allgemeine oder bevorzugte Bedeutung hat.

Ferner betrifft die Erfindung bevorzugt eine Verbindung der Formel (I) gekennzeichnet durch eine Verbindung der Formel (II),

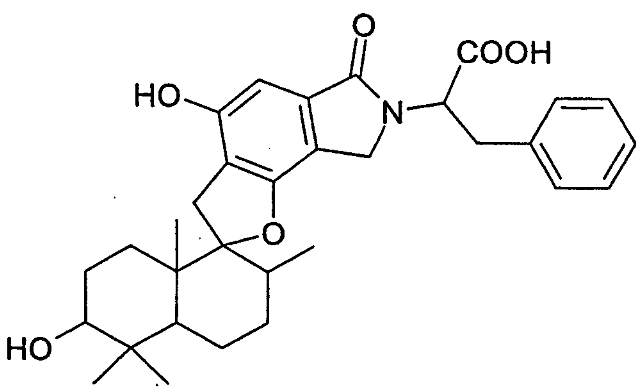

(II)

eine Verbindung der Formel (III) oder

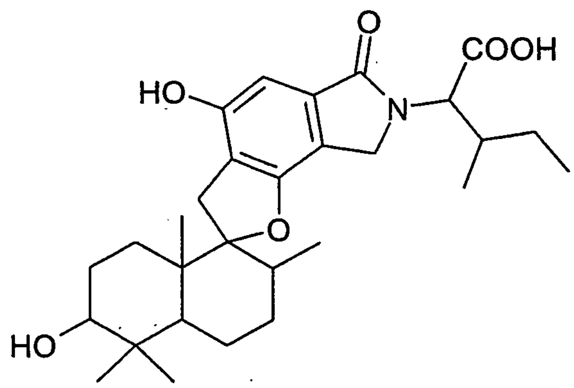

(III)

eine Verbindung der Formel (IV)

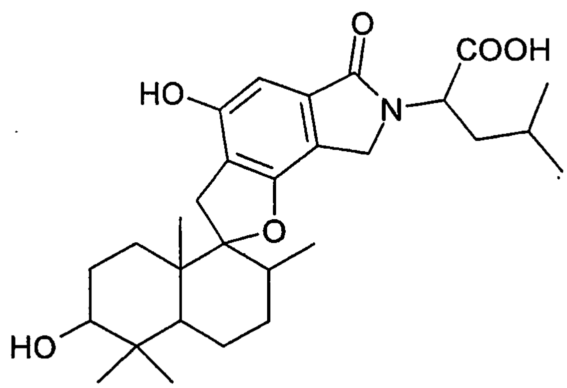

(IV)

oder ein physiologisch verträgliches Salz davon.

Chiralitätszentren in den Verbindungen der Formel (I), (II), (III) und (IV) können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diasteromerengemische.

$C_1$-$C_6$-Alkyl bedeutet ein geradkettiges oder verzweigtes Alkyl mit 1 bis 6 C-Atomen, bevorzugt mit 1 bis 4 C-Atomen, z.B. Methyl, Ethyl, i-Propyl, tert.Butyl und Hexyl.

$C_2$-$C_6$-Alkenyl bedeutet ein geradkettiges oder verzweigtes Alkenyl mit 2 bis 6 C-Atomen, das einfach, zweifach oder dreifach ungesättigt ist, z.B. Allyl, Crotyl, 1-Propenyl, Penta-1,3-dienyl und Pentenyl.

$C_2$-$C_6$-Alkinyl bedeutet ein geradkettiges oder verzweigtes Alkinyl mit 2 bis 6 C-Atomen, das einfach oder zweifach ungesättigt ist, z.B. Propinyl, Butinyl und Pentinyl.

$C_5$-$C_{14}$-Aryl bedeutet eine Arylgruppe mit 5 bis 14 C-Atomen, z.B. Phenyl, Benzyl oder 1- oder 2- Naphthyl, die substituiert oder mit ein, zwei oder drei Substituenten aus der Gruppe Halogen, z.B. Chlor, Brom, Fluor, $C_1$-$C_4$-Alkyl, z.B. Methyl, Hydroxy, $C_1$-$C_4$-Alkoxy, z.B. Methoxy oder mit Trifluormethyl unsubstituiert sind.

Halogen bedeutet ein Element aus der Gruppe Fluor, Chlor, Brom oder lod.

Die Erfindung betrifft ferner ein Verfahren zur Herstellung einer Verbindung der Formel (I), dadurch gekennzeichnet, daß der Stamm Stachybotris atra ST002348, DSM 14952, oder einer seiner Varianten oder Mutanten unter geeigneten Bedingungen in einem Kulturmedium fermentiert wird, bis sich eine oder mehrere der Verbindungen der Formel (I) in dem Kulturmedium anhäufen und anschließend aus dem Kulturmedium isoliert und gegebenenfalls in chemische Äquivalente und/oder ein physiologisch verträgliches Salz umgewandelt wird.

Vorzugsweise wird der Stamm Stachybotris atra ST002348, DSM 14952, seine Mutanten und/oder Varianten in einer Nährlösung oder einem Festmedium (auch als Kulturmedium bezeichnet) mit Kohlenstoff- und Stickstoffquelle sowie den üblichen anorganischen Salzen fermentiert, bis sich die erfindungsgemäßen Verbindungen in dem Kulturmedium anhäufen, anschließend werden die Verbindungen aus dem Kulturmedium isoliert und gegebenenfalls in die einzelnen aktiven Komponenten aufgetrennt.

Das erfindungsgemäße Verfahren kann für die Fermentation im Labormaßstab (Milliliter- bis Literbereich) und für den industriellen Maßstab (Kubikmetermaßstab) eingesetzt werden.

Eine stark produzierende Kolonie von Stachybotris atra wurde in einer Vorkultur vermehrt. Der Stamm wurde bei der Deutschen Sammlung von Mikroorganismen und Zellkulturen GmbH, Mascheroder Weg 1 B, 3300 Braunschweig, Deutschland, nach den Regeln des Budapester Vertrages am 23.04.2002 unter der Nummer DSM 14952 bzw. dem vom Hinterleger zugeteiltem Bezugszeichen ST002348 von Aventis Pharma Deutschland GmbH hinterlegt.

Stachybotris atra ST002348, DSM 14952, besitzt auf Malz-Agar ein weiß bis orangefarbenes Mycel. Der Stamm bildet für die Art charakteristische hyaline ellipsoide warzige Konidien (8-11 x 6-11 $\mu$m).

Anstelle des Stammes Stachybotris atra ST002348, DSM 14952, können auch dessen Mutanten und Varianten eingesetzt werden, die ein oder mehrere der erfindungsgemäßen Verbindungen synthetisieren.
Eine Mutante ist ein Mikroorganismus, in dem ein oder mehrere Gene des Genoms modifiziert wurden, wobei das Gen oder die Gene funktionell und vererbbar erhalten bleiben, die für die Fähigkeit des Organismus verantwortlich sind, die erfinderische Verbindung zu produzieren.

Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS); 2-Hydroxy-4-methoxy-benzophenon (MOB) oder N-Methyl-N'-nitro-N-nitrosoguanidin (MNNG) erzeugt werden, oder wie von Brock et al. in "Biology of Microorganisms", Prentice Hall, Seite 238-247 (1984) beschrieben.

Eine Variante ist ein Phenotyp des Mikroorganismus. Mikroorganismen haben die Fähigkeit, sich an ihre Umgebung anzupassen und zeigen daher ausgeprägte physiologische Flexibilität. Bei der phenotypischen Anpassung sind alle Zellen des Mikroorganismus involviert, wobei die Art der Veränderung nicht genetisch konditioniert ist und unter veränderten Bedingungen reversibel ist (H. Stolp, Microbial ecology: organismus, habitats, activities. Cambridge University Press, Cambridge, GB, Seite 180, 1988).

Das Screening nach Mutanten und Varianten, die ein oder mehrere der erfindungsgemäßen Verbindungen synthetisieren, erfolgt nach folgendem Schema:

- Herstellen von Mutanten und/oder Varianten nach ansich bekannten Methoden;
- Kultivieren der dabei erhaltenen Mutante und/oder Variante;
- Lyophilisierung der Schüttelkulturen;
- Extraktion des Lyophilisats mit einem organischen Lösungsmittel;
- Extraktion der Verbindung aus dem Kulturfiltrat mit Festphasen;
- Analytik mittels HPLC, DC oder durch Testen der biologischen Wirksamkeit;
- optional Aufklärung der Taxonomie der Mutante und/oder Variante.

Die im folgenden beschriebenen Fermentationsbedingungen gelten für Stachybotris atra ST002348, DSM 14952, sowie Mutanten und Varianten davon.

In einer Nährlösung, die eine Kohlenstoffquelle und eine Stickstoffquelle sowie die üblichen anorganischen Salze enthält, produziert Stachybotris atra ST002348, DSM 14952, die Spirobenzofuranlactam-Derivate.

Als bevorzugte Kohlenstoffquellen für die aerobe Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt oder Stärke. Als stickstoffhaltige Nährstoffe kommen in Betracht: Aminosäuren; Peptide; Proteine; Abbauprodukte von Proteinen und Peptiden, insbesondere synthetisch bzw. biosynthetisch gewonnene Peptide, beispielsweise Casein, Peptone oder Tryptone; Fleischextrakte; Hefeextrakte; gemahlene Samen, beispielsweise von Mais, Weizen, Bohnen, Soja oder der Baumwollpflanze; Destillationsrückstände der Alkoholherstellung; Fleischmehle oder Hefeextrakte; Ammoniumsalze; Nitrate. Übliche anorganische Salze sind beispielsweise Chloride, Carbonate, Sulfate oder Phosphate eines Alkali- oder Erdalkalimetalles, von Eisen, Zink, Kobalt oder Mangan.

Die Bildung der erfindungsgemäßen Verbindungen verläuft besonders gut in einer Nährlösung, die etwa 0,1 bis 5 %, bevorzugt 0,5 bis 2 % Stärke, 0,2 bis 5 %, bevorzugt 0,5 bis 1 % Hefeextrakt und 0,2 bis 5 %, bevorzugt 0,5 bis 2 % Glucose enthält. Die Angaben in Prozent sind jeweils bezogen auf das Gewicht der gesamten Nährlösung.

In dieser Nährlösung bildet Stachybotris atra ST002348, DSM 14952, ein Gemisch aus Spirobenzofuranlactam-Derivaten. Je nach Zusammensetzung der Nährlösung kann der mengenmäßige Anteil eines oder mehrerer der erfindungsgemäßen Spirobenzofuranlactam-Derivate variieren.

Die Kultivierung des Mikroorganismus erfolgt aerob, also beispielsweise submers unter Schütteln oder Rühren in Schüttelkolben oder Fermentern gegebenenfalls unter Einführen von Luft oder Sauerstoff. Sie kann in einem Temperaturbereich von etwa 18 bis 35˚C, vorzugsweise bei etwa 20 bis 30˚C, insbesondere bei 22 bis 27˚C durchgeführt werden. Der pH-Bereich sollte zwischen 4 und 8 liegen, vorzugsweise zwischen 5 und 6. Man kultiviert den Mikroorganismus unter diesen Bedingungen im allgemeinen über einen Zeitraum von 48 bis 200 Stunden, vorzugsweise 72 bis 168 Stunden.

Vorteilhaft kultiviert man in mehreren Stufen, d. h. man stellt zunächst eine oder mehrere Vorkulturen in einem

flüssigen Nährmedium her, die dann in das eigent-liche Produktionsmedium, die Hauptkultur, beispielsweise im Volumenverhältnis 1:10 bis 1:100, überimpft werden. Die Vorkultur erhält man, z.B., indem man ein Mycel in eine Nährlösung überimpft und etwa 36 bis 120 Stunden, bevorzugt 48 bis 72 Stunden, wachsen läßt. Das Mycel kann beispielsweise erhalten werden, indem man den Stamm etwa 3 bis 21 Tage, bevorzugt 4 bis 10 Tage, auf einem festen oder flüssigen Nährboden, beispielsweise Malz-Hefe-Agar oder Kartoffel-Dextrose-Agar wachsen läßt.

Der Fermentationsverlauf kann anhand des pH-Wertes der Kulturen oder des Mycelvolumens sowie durch chromatographische Methoden, wie z.B. Hochleistungsflüssigchromatographie (HPLC), oder Ausprüfen der biologischen Aktivität überwacht werden.

Das im folgenden beschriebene Isolierungsverfahren dient zur Aufreinigung der erfindungsgemäßen Spirobenzofuranlactam-Derivate der Formel (I):

Die Isolierung bzw. Aufreinigung eines erfindungsgemäßen Spirobenzofuranlactam-Derivates aus dem Kulturmedium erfolgt nach bekannten Methoden unter Berücksichtigung der chemischen, physikalischen und biologischen Eigenschaften der Naturstoffe. Zum Testen der Konzentration der jeweiligen Spirobenzofuranlactam-Derivate im Kulturmedium oder in den einzelnen Isolierungsstufen kann die HPLC verwendet werden, wobei die Menge der gebildeten Substanz zweckmäßig mit einer Eichlösung verglichen wird.

Zur Isolierung werden die Kulturbrühe oder die Kultur samt Festmedium lyophilisiert, anschließend werden die Spirobenzofuranlactam-Derivate vom Lyophilisat mit einem gegebenenfalls mit Wasser mischbaren organischen Lösungsmittel extrahiert. Die organische Lösungsmittelphase enthält die erfindungsgemäßen Naturstoffe, sie wird gegebenenfalls im Vakuum konzentriert und weiter aufgereinigt.

Die weitere Aufreinigung einer oder mehrerer erfindungsgemäßer Verbindungen erfolgt durch Chromatographie an geeigneten Materialien, vorzugsweise z.B. an Molekularsieben, an Kieselgel, Aluminiumoxid, an Ionenaustauschern oder an Adsorberharzen bzw. an Umkehrphasen (reversed phase, RP). Mit Hilfe dieser Chromatographie werden die Spirobenzofuranlactam-Derivate getrennt. Die Chromatographie der Spirobenzofuranlactam-Derivate erfolgt mit gepufferten wässrigen Lösungen oder Gemischen von wässrigen und organischen Lösungen.

Unter Gemischen von wässrigen oder organischen Lösungen versteht man alle mit Wasser mischbaren organischen Lösemittel, vorzugsweise Methanol, 2-Propanol und Acetonitril, in einer Konzentration von 5 bis 80 % Lösemittel, vorzugsweise 20 bis 50 % Lösemittel oder auch alle gepufferten wässrigen Lösungen, die mit organischen Lösemitteln mischbar sind. Die zu verwendenden Puffer sind die gleichen wie oben angegeben.

Die Trennung der Spirobenzofuranlactam-Derivate aufgrund ihrer unterschiedlichen Polarität erfolgt mit Hilfe der Reversed Phase Chromatographie, beispielsweise an MCI® (Adsorberharz von Mitsubishi, Japan) oder Amberlite XAD® (TOSOHAAS), oder an weiteren hydrophoben Materialien, wie zum Beispiel an RP-8- oder RP-18-Phasen. Außerdem kann die Trennung mit Hilfe der Normalphasen-Chromatographie, zum Beispiel an Kieselgel, Aluminiumoxid und ähnlichen erfolgen.

Die Chromatographie der Spirobenzofuranlactam-Derivate erfolgt mit gepufferten oder angesäuerten wäßrigen Lösungen oder Gemischen von wäßrigen Lösungen mit Alkoholen oder anderen, mit Wasser mischbaren organischen Lösemitteln. Als organisches Lösemittel wird vorzugsweise 2-Propanol und Acetonitril verwendet.

Unter gepufferten oder angesäuerten wäßrigen Lösungen versteht man z.B. Wasser, Phosphatpuffer, Ammoniumacetat, Citratpuffer in einer Konzentration von bis zu 0,5 M sowie Ameisensäure, Essigsäure, Trifluoressigsäure oder allen handelsüblichen, dem Fachmann bekannten Säuren, vorzugsweise in einer Konzentration von bis zu 1 %. Bei gepufferten wäßrigen Lösungen wird 0,1 % Ammoniumacetat besonders bevorzugt.

Chromatographiert wurde mit einem Gradienten, der mit 100 % Wasser beginnt und mit 100 % Lösemittel endet, vorzugsweise wurde ein linearer Gradient von 20 bis 100 % 2-Propanol oder Acetonitril gefahren.

Alternativ kann auch eine Gelchromatographie oder die Chromatographie an hydrophoben Phasen erfolgen. Die Gelchromatographie wird an Polyacrylamid- oder Mischpolymergelen durchgeführt, wie z.B. Biogel-P 2® (Fa. Biorad) oder Fractogel TSK HW 40® (Fa. Merck, Deutschland oder Toso Haas, USA). Die Reihenfolge der vorgenannten Chromatographien ist umkehrbar.

Die vorliegende Erfindung betrifft weiterhin alle offensichtlichen chemischen Äquivalenten der erfindungsgemäßen Verbindungen der Formel (I). Solche Äquivalente sind Verbindungen, die einen geringfügigen chemischen Unterschied aufweisen, also die gleiche Wirkung haben oder sich unter milden Bedingungen in die erfindungsgemäßen Verbindungen umwandeln. Zu den genannten Äquivalenten gehören z.B. auch Salze, Reduktionsprodukte, Oxidationsprodukte, Ester, Ether, Acetale oder Amide der Verbindungen der Formel (I) sowie Äquivalente die der Fachmann mit Standardmethoden herstellen kann, darüber hinaus alle optische Antipoden, Diastereomere und alle stereomere Formen.

Unter physiologisch verträglichen Salzen von Verbindungen der Formel (I) versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Ester, Ether, Amide und Acetale können nach literaturbeschriebenen Methoden hergestellt werden, z.B. in Advanced Organic Synthesis, 4th Edition, J. March, John Wiley & Sons, 1992 oder Protective Groups in Organic Synthesis 3rd Edition, T. W. Greene & P. G. M. Wuts, John Wiley & Sons, 1999.

Die Carboxylgruppe kann beispielsweise mit $LiAlH_4$ zum Alkohol reduziert werden oder unter Zugabe katalytischer Mengen einer anorganischen Säure (beispielsweise $H_2SO_4$ oder HCl) verestert werden. Die Hydroxygruppen können beispielsweise unter den Bedingungen der Williamson'schen Ethersynthese verethert werden.

Zum Nachweis der Inhibitoren von PAI-1 bedient man sich eines Testes, bei dem die Aktivierung eines spezifischen Substrates durch tPA in Anwesenheit einer definierten Menge PAI-1 und der jeweils zu untersuchenden Substanz gemessen wird. tPA wird durch PAI-1 inhibiert. Eine Hemmung von PAI-1 wirkt sich in einer erhöhten tPA-Aktivität aus. Die enzymatische Aktivität von tPA wird colorimetrisch vermessen, indem ein chromogenes Substrat eingesetzt wird, welches sich nach Amidolyse färbt.

$IC_{50}$-Werte für die Spirobenzofuranlactam-Derivate sind in der Tabelle 1 angegeben:

| | $IC_{50}$-Wert |
|---|---|
| Verbindung der Formel (II) | 41 $\mu$M |
| Verbindung der Formel (III) | 66 $\mu$M |
| Verbindung der Formel (IV) | 35 $\mu$M |

Als Inhibitoren von PAI-1 können die erfindungsgemäßen Verbindungen zur Behandlung und/oder Prophylaxe der einleitend genannten Erkrankungen verwendet werden.
Gegenstand der Erfindung ist daher ferner die Verwendung einer erfindungsgemäßen Verbindung der Formel (I) oder eines physiologisch verträglichen Salzes derselben als Arzneimittel in der Human- oder Tiermedizin bzw. zur Herstellung eines Arzneimittels in der Human- oder Tiermedizin, insbesondere zur Behandlung und/oder Prophylaxe von koronarer Herzerkrankung, akutem Myokardinfarkt, instabiler Angina pectoris, Venenthrombose und venösen Thromboembolien, arteterieller Thrombose, Arteriosklerose, Insulinresistenz und makrovaskulären Verletzungen bei Typ-II Diabetes mellitus-Patienten, Hypoxie, septischem Schock, Lungenentzündung und Lungenfibrose, sowie Krebserkrankungen, insbesondere Brustkrebs, Darmkrebs, Magenkrebs, Leberkrebs, Gehirntumoren, Ovarialtumoren, Speiseröhrenkrebs, Nierenkrebs, Muskelzellkarzinom, insbesondere Kopf- und Nackenmuskelkarzinom, besonders bevorzugt eines Arzneimittels zur Gerinnungshemmung zur Behandlung von und/oder als Prophylaxe für thromboembolische Erkrankungen.

Des weiteren betrifft die vorliegende Erfindung ein Arzneimittel mit einem Gehalt an mindestens einer Verbindung der Formel (I), wobei die Verbindung oder die Verbindungen der Formel (I) grundsätzlich als solche in Substanz verabreicht werden kann oder bevorzugt in Mischung mit einem oder mehreren der üblichen pharmakologisch geeigneten Träger- oder Hilfsstoffen.

Die erfindungsgemäßen Verbindungen sind im festen Zustand und in Lösungen im pH-Bereich zwischen 3 und 8, insbesondere 5 und 7, stabil und lassen sich damit in übliche galenische Zubereitungen einarbeiten.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden.

Übliche pharmakologisch geeignete Träger- oder Hilfsstoffe sind beispielsweise Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Spirobenzofuranlactam-Derivate enthält. Bei festen Dosierungseinheiten wie Tabletten, Kapseln und Suppositorien kann diese Dosis bis zu etwa 500 mg, bevorzugt jedoch etwa 0,1 bis 200 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung

der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, daß man eine oder mehrere der erfindungsgemäßen Verbindungen der Formel (I) optional mit einem oder mehreren der üblichen Träger- oder Hilfsstoffe in eine geeignete Darreichungsform bringt.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

Beispiel 1: Herstellung einer Glycerinkultur von Stachybotris atra ST002348, DSM 14952

100 mL Nährlösung (Malzextrakt 2,0%, Hefeextrakt 0,2 %, Glukose 1,0% $(NH_4)_2HPO_4$ 0,05 %, pH 6,0) wurden in einem sterilen 300 mL Erlenmeyerkolben mit dem Stamm Stachybotris atra ST002348, DSM 14952, beimpft und 6 Tage bei 25˚ C und 140 UpM auf einer rotierenden Schüttelmaschine inkubiert. 1,5 mL dieser Kultur wurden anschließend mit 2,5 mL 50 %igem Glycerin verdünnt und bei -135˚C gelagert.

Beispiel 2: Herstellung einer Vorkultur im Erlenmeyerkolben von Stachybotris atra ST002348, DSM 14952

Ein 300 mL Erlenmeyerkolben mit 100 mL der folgende Nährlösung: Malzextrakt 2,0 %, Hefeextrakt 0,2 %, Glucose 1,0 % $(NH_4)_2HPO_4$ 0,05 %, pH 6,0 wurde mit einer auf einem Schrägröhrchen/Petrischale (gleiche Nährlösung, jedoch mit 2 % Agar) gewachsenen Kultur oder mit 1 mL einer Glycerinkultur (s. Beispiel 1) beimpft und auf einer Schüttelmaschine bei 140 UpM und 25 ˚C inkubiert.

Beispiel 3: Herstellung einer Hauptkultur im Erlenmeyerkolben von Stachybotris atra ST002348, DSM 14952

Ein 300 mL Erlenmeyerkolben mit 100 mL der folgende Nährlösung: 0,5 % lösliche Stärke, 0,5 % Maisstärke, 1 % Glucose, 0,5 % Hefeextrakt, 0.5% "cornsteep" und 0.2% $CaCO_3$, wurde mit 2 mL einer Vorkultur (s. Beispiel 2) beimpft und auf einer Schüttelmaschine bei 140 UpM und 25˚ C inkubiert. Die maximale Produktion einer oder mehrerer Verbindungen der erfindungsgemäßen Spirobenzofuranlactam-Derivate ist nach ca. 120 Stunden erreicht. Zum Animpfen von 10 L Fermentern genügte eine 48 bis 96 Std. alte Submerskultur (Animpfmenge ca. 10 %) aus der gleichen Nährlösung.

Beispiel 4: Herstellung der Spirobenzofuranlactam-Derivate

Ein 30 L Fermenter wurde unter folgenden Bedingungen betrieben:

| | |
|---|---|
| Nährmedium: | 5 g/L Stärke |
| | 5 g/L Maisstärke |
| | 5 g/L Cornsteep flüssig |
| | 5 g/L Hefeextrakt |
| | 5 g/L $CaCO_3$ |
| | pH 6,0 (vor der Sterilisation) |
| Inkubationszeit: | 96 Stunden |
| Inkubationstemperatur: | 25˚ C |
| Rührergeschwindigkeit: | 150 UpM |
| Belüftung: | 15 L $Min^{-1}$ |

Durch wiederholte Zugabe von ethanolischer Polyollösung konnte die Schaumbildung unterdrückt werden. Das Produktionsmaximum wurde nach ca. 72 bis 120 Stunden erreicht.

Beispiel 5: Isolierung der Spirobenzofuranlactam-Derivate aus den Schüttelkulturen von Stachybotris atra ST002348, DSM 14952

Nach Beendigung der Fermentation Stachybotris atra ST002348, DSM 14952 wurde die Kulturbrühe von 50 Schüttelkolben (jeweils 100 mL Kulturbrühe) mitsamt der Biomasse lyophilisiert und das Lyophilisat mit 2 x 5 Liter Methanol extrahiert. Die wirkstoffhaltige, methanolische Lösung wurde durch Filtration vom Rückstand befreit und im

Vakuum konzentriert. Das Konzentrat wurde mit Wasser verdünnt und auf eine vorbereitete 1,0 Liter MCI GEL, CHP20P-Säule aufgetragen. Eluiert wurde mit einem Gradienten von Wasser nach 100% Acetonitril in 60 Minuten. Der Säulendurchfluß (30 mL pro Minute) wurde fraktioniert aufgefangen (je 30 mL) und die Spirobenzofuranlactam-Derivate enthaltenden Fraktionen (Fr. 26-40) zusammengefaßt. Nach Konzentrieren im Vakuum und anschließender Lyophilisierung wurden 2,9 g eines hellrosa Pulvers isoliert.

Beispiel 6: Vortrennung der Spirobenzofuranlactam-Derivate durch RP18-Chromatographie.

Circa 1 g des nach Beispiel 5 gewonnenen Produktes wurden auf eine LiChrospher® 100 RP-18 (e) Säule (Größe: 50 mm x 250 mm; Fa. Merck, Darmstadt) aufgetragen und mit einem Gradienten von 20 % Acetonitril (+ Wasser mit 0,1 % Trifluoressigsäure-Zusatz) nach 90 % Acetonitril mit einer Flußrate von 45 mL pro Minute eluiert. Der Säulenausfluß wurde fraktioniert (45 mL) aufgefangen, wobei sich hauptsächlich in den Fraktionen 95 bis 112 Spirobenzofuranlactam-Derivate befanden. Sie wurden zusammengefaßt, im Vakuum vom Lösungsmittel befreit und anschließend lyophilisiert. Die Ausbeute betrug 170 mg.

Beispiel 7: Reinigung der Spirobenzofuranlactam-Derivate.

100 mg des nach Beispiel 6 isolierten und angereicherten Gemisches wurden auf eine LUNA® 5µ C18(2) Säule (Größe: 21 mm x 250 mm) aufgetragen und mit einem Gradienten von 30 bis 85 % Acetonitril (+ Wasser mit 0,05 % TFA-Zusatz) chromatographiert. Der Durchfluß des Elutionsmittels betrug 25 mL pro Minute, die Fraktionsgröße 25 ml. Fraktion 32 enthielt die Verbindung der Formel (II) und ergab nach Lyophilisieren 6,1 mg. Fraktion 33 enthielt die Verbindung der Formel (III) und ergab nach Lyophilisieren 5,5 mg. Fraktion 34 enthielt die Verbindung (IV) und ergab nach Lyophilisieren 5,2 mg.

Die physikalisch-chemischen sowie spektroskopischen Eigenschaften der erfindungsgemäßen Substanzen lassen sich wie folgt zusammenfassen:

Beispiel 8: Charakterisierung der Verbindung der Formel (II):

Summenformel: $C_{32}H_{39}NO_6$
Molekulargewicht: 533,67
UV-Maxima: 248, 348

Tabelle 2: $^1H$- und $^{13}C$-chemische Verschiebungen von Verbindung (II) in DMSO-$d_6$ bei 300K

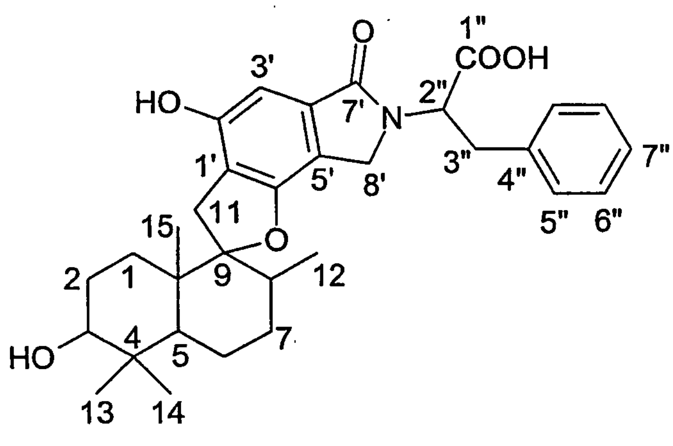

(II)

| | $^1H$ | $^{13}C$ a) |
|---|---|---|
| 1 | 1.73/0.92 | 23.8 |
| 2 | 1.81/1.40 | 24.8 |

(fortgesetzt)

| | $^1H$ | $^{13}C$ a) |
|---|---|---|
| 3 | 3.18 | 73.3 |
| 5 | 2.01 | 39.3 |
| 6 | 1.46/1.40 | 20.4 |
| 7 | 1.53/1.39 | 30.7 |
| 8 | 1.77 | 36.4 |
| 11 | 3.1-0/2.73 | 31.6 |
| 12 | 0.59 | 15.5 |
| 13 | 0.88 | 28.6 |
| 14 | 0.79 | 22.3 |
| 15 | 0.94 | 15.8 |
| 2'-OH | 9.68 | - |
| 3' | 6.49 | 100.8 |
| 8' | 4.26 | 44.3 |
| 2" | 5.11 | 54.5 |
| 3" | 3.33/3.27 | 34.4 |
| 5" | 7.22 | 128.2 |
| 6" | 7.25 | 128.4 |
| 7" | 7.14 | 126.4 |
| a) Die Werte für die $^{13}C$-chemischen Verschiebungen werden nur auf eine Nachkommastelle angegeben, da sie aus dem HMQC-Spektrum bestimmt wurden. | | |

Beispiel 9: Charakterisierung der Verbindung der Formel (III):

Summenformel: $C_{29}H_{41}NO_6$
Molekulargewicht: 499,65
UV-Maxima: 248, 348

Tabelle 3: $^{1}H$- und $^{13}C$-chemische Verschiebungen von Verbindung (III) in DMSO-$d_6$ bei 300K

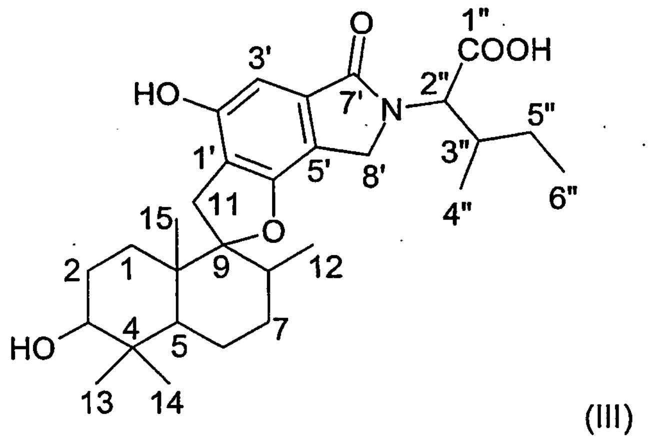

(III)

| | $^{1}H$ | $^{13}C$ a) |
|---|---|---|
| 1 | 1.74/0.92 | 23.8 |
| 2 | 1.79/1.39 | 24.8 |
| 3 | 3.18 | 73.4 |
| 3-OH | 4.07 | - |
| 4 | - | 37.3 |
| 5 | 2.03 | 39.4 |
| 6 | 1.44 | 20.4 |
| 7 | 1.52/1.41 | 30.7 |
| 8 . | 1.79 | 36.5 |
| 9 | - | 98.0 |
| 10 | - | 41.8 |
| 11 | 3.12/2.77 | 31.6 |
| 12 | 0.66 | 15.5 |
| 13 | 0.88 | 28.6 |
| 14 | 0.80 | 22.3 |
| 15 | 0.95 | 15.7 |
| 1' | - | 117.0 |
| 2' | - | 153.8 |
| 2'-OH | 9,73 | - |
| 3' | 6.58 | 100.9 |
| 4' | - | 132.8 |
| 5' | - | 112.2 |
| 6' | - | 155.8 |
| 7' | - | 167.9 |
| 8' | 4.39/4.28 | 44.2 |
| 1" | - | 172.1 |

(fortgesetzt)

| | $^1H$ | $^{13}C$ a) |
|---|---|---|
| 2" | 4.55 | 58.3 |
| 3" | 2.12 | 33.8 |
| 4" | 0.96 | 15.7 |
| 5" | 1.31/1.05 | 25.1 |
| 6" | 0.84 | 10.3 |
| a) Die Werte für die $^{13}C$-chemischen Verschiebungen werden nur auf eine Nachkommastelle angegeben, da sie aus den 2D-Spektren bestimmt wurden. | | |

Beispiel 10: Charakterisierung der Verbindung der Formel (IV):

Summenformel: $C_{29}H_{41}NO_6$
Molekulargewicht: 499,65
UV-Maxima: 248, 348

Tabelle 4: $^1H$- und $^{13}C$-chemische Verschiebungen von Verbindung (IV) in DMSO-$d_6$ bei 300K

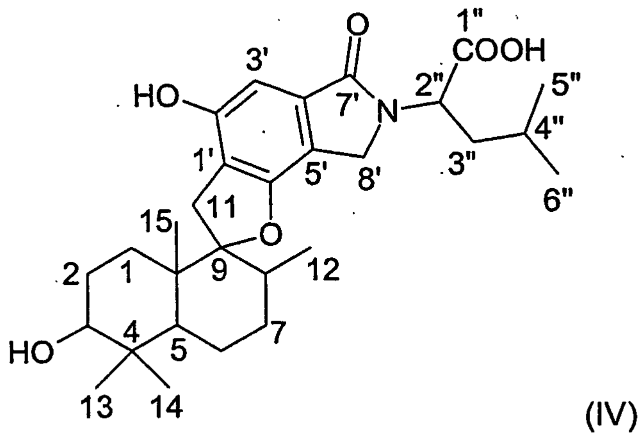

(IV)

| | $^1H$ | $^{13}C$ |
|---|---|---|
| 1 | 1.75/0.94 | 23.80 |
| 2 | 1.80/1.41 | 24.77 |
| 3 | 3.18 | 73.38 |
| 3-OH | 4.09 | - |
| 4 | - | 37.22 |
| 5 | 2.03 | 39.32 |
| 6 | 1.41 | 20.37 |
| 7 | 1.52/1.41 | 30.65 |
| 8 | 1.79 | 36.47 |
| 9 | - | 97.96 |

(fortgesetzt)

| | $^1H$ | $^{13}C$ |
|---|---|---|
| 10 | - | 41.72 |
| 11 | 3.13/2.77 | 31.63 |
| 12 | 0.65 | 15.48 |
| 13 | 0.88 | 28.53 |
| 14 | 0.79 | 22.29 |
| 15 | 0.95 | 15.75 |
| 1’ | - | 116.85 |
| 2’ | - | 153.68 |
| 2’-OH | 9.72 | - |
| 3’ | 6.58 | 100.83 |
| 4’ | - | 133.12 |
| 5’ | - | 112.22 |
| 6’ | - | 155.85 |
| 7’ | - | 168.03 |
| 8’ | 4.30/4.25 | 43.77 |
| 1" | - | 173.03 |
| 2" | 4.81 | 51.50 |
| 3" | 1.98/1.70 | 37.41 |
| 4" | 1.40 | 24.50 |
| 5" | 0.91 | 22.82 |
| 6" | 0.87 | 20.78 |

Beispiel 11: Bioassay auf PAI-1 Inhibitoren

Reaktion: Die Inhibierung der enzymatischen Aktivität von tPA durch PAI-1 wird durch die Amidolyse des chromogenen Substrates H-D-Ile-Pro-Arg-pNA (Fa. Chromogenix; pNA = para-Nitroanilin) als optische Dichte (OD) bei einer Wellenlänge von 405 nm gemessen.

Testsubstanzen:

Extrakte oder Reinsubstanzen, beispielsweise die in den Beispielen 4-10 hergestellten bzw. charakterisierten Spirobenzofuranlactam-Derivate, die in DMSO gelöst vorliegen, wurden in geeigneter Weise mit TRIS-Puffer pH 8.4 verdünnt.

Methode:

5 µl Testsubstanz und 5 µl PAI-1 werden 30 Minuten bei Raumtemperatur vorinkubiert. Anschliessend werden 10 µl tPA-Lösung und 20 µl Substrat-Lösung zugesetzt. Die Endkonzentrationen in der Probe betragen 50 µM Testsubstanz, 4,5 nM PAI-1, 7,5 nM tPA und 1 mM Substrat in TRIS-Puffer pH 8.4. Unmittelbar nach Zusatz des Substrats wird die Anfangsabsorption bei 405 nm gemessen. Nach 60 Minuten Inkubation bei 37° C wird die Absorption erneut gemessen.
Es werden jeweils Blank-Proben (Puffer anstelle von tPA), Positiv-Kontrollen. = B (Puffer anstelle von Testsubstanzen) und tPA-Kontrollen = A (Puffer anstelle von PAI-1) mitgetestet.
Nach Korrektur mittels der Blank-Proben wird die Inhibierung nach der folgenden Gleichung ermittelt:

$$\%Inhibition = 100 - \frac{\Delta OD_{405nm}\ Mittelwert\ A - \Delta OD_{405nm}\ Probe}{\Delta OD_{405nm}\ Mittelwert\ A - \Delta OD_{405nm}\ Mittelwert\ B} * 100$$

Die Ergebnisse des Assays sind als $IC_{50}$-Werte in Tabelle 1 wiedergegeben.

## Patentansprüche

1. Verbindung der Formel (I)

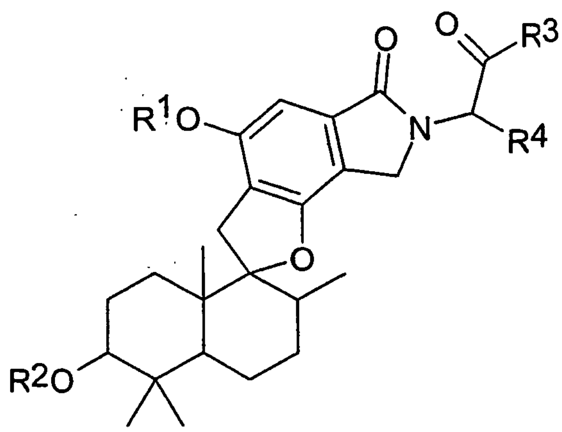

(I)

wobei
$R^1$ und $R^2$ unabhängig voneinander H, $C_1$-$C_6$-Alkyl, $C_2$-$C_6$-Alkenyl, $C_2$-$C_6$-Alkinyl oder $C_5$-$C_{14}$-Aryl bedeutet, worin Alkyl, Alkenyl, Alkinyl und Aryl unsubstituiert oder ein- bis dreifach durch einen Rest aus der Gruppe -OH, =O, -O-$C_1$-$C_6$-Alkyl, -O-$C_2$-$C_6$-Alkenyl, -O-$C_5$-$C_{14}$-Aryl, -NH-$C_1$-$C_6$-Alkyl, -NH-$C_2$-$C_6$-Alkenyl, -NH[-C(=O)-($C_1$-$C_6$-Alkyl)], -NH[-C(=O)-($C_5$-$C_{14}$-Aryl)], -$NH_2$ oder Halogen substituiert sind,
$R^3$ -OH, -O-$R^1$ oder -NH-$R^1$ bedeutet, und
$R^4$ H, $C_1$-$C_6$-Alkyl, $C_2$-$C_6$-Alkenyl, $C_5$-$C_{14}$-Aryl oder -($C_1$-$C_6$-Alkyl)-($C_5$-$C_{14}$-Aryl) bedeutet,
oder ein physiologisch verträgliches Salz der Verbindung der Formel (I).

2. Verbindung gemäß Anspruch 1, wobei $R^1$ und $R^2$ unabhängig voneinander gleich H oder $C_1$-$C_6$-Alkyl sind, bevorzugt H.

3. Verbindung gemäß einem der Ansprüch 1 oder 2, wobei $R^3$ gleich OH oder -O-($C_1$-$C_6$-Alkyl) ist, bevorzugt OH.

4. Verbindung gemäß einem der Ansprüch 1 bis 3, wobei $R^4$ gleich $C_1$-$C_6$-Alkyl oder -($C_1$-$C_6$-Alkyl)-($C_5$-$C_{14}$-Aryl), bevorzugt Benzyl, 2-Butyl oder 1-(2-Methyl-propyl).

5. Verbindung gemäß einem der Ansprüch 1 bis 4, **dadurch gekennzeichnet, dass** die Verbindung der Formel (I) die folgende Formel (II) aufweist

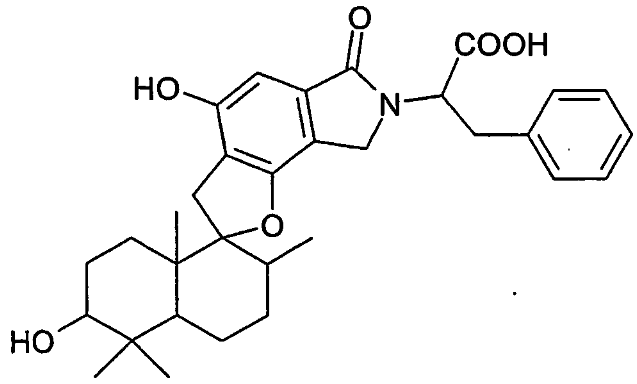

(II).

6. Verbindung gemäß einem der Ansprüch 1 bis 4, **dadurch gekennzeichnet, dass** die Verbindung der Formel (I) die folgende Formel (III) aufweist

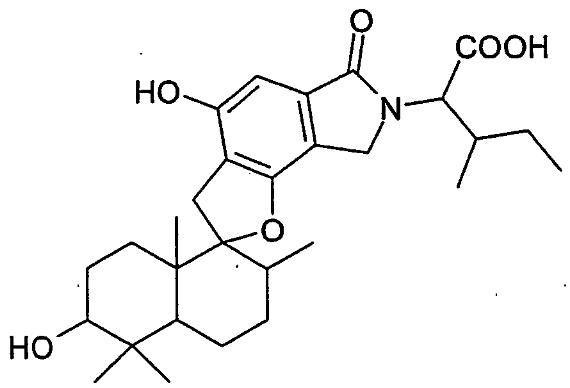

(III).

7. Verbindung gemäß einem der Ansprüch 1 bis 4, **dadurch gekennzeichnet, dass** die Verbindung der Formel (I) die folgende Formel (IV) aufweist

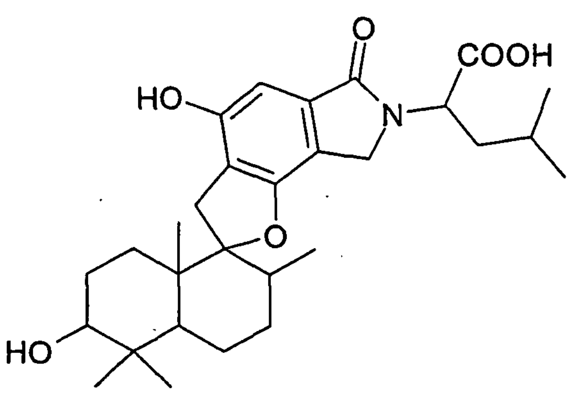

(IV).

8. Verfahren zur Herstellung einer Verbindung der Formel (I) gemäß einem der Ansprüche 1 bis 7, **dadurch gekennzeichnet, daß** der Stamm Stachybotris atra ST002348, DSM 14952, oder einer seiner Varianten oder Mutanten unter geeigneten Bedingungen in einem Kulturmedium fermentiert wird, bis sich eine oder mehrere der Verbindungen der Formel (I) in dem Kulturmedium anhäufen und anschließend aus dem Kulturmedium isoliert und gegebenenfalls

in ein physiologisch verträgliches Salz umgewandelt wird.

**9.** Verwendung einer Verbindung der Formel (I) oder eines physiologisch verträglichen Salzes derselben gemäß einem oder mehrerer der Ansprüche 1 bis 7 zur Herstellung eines Arzneimittel.

**10.** Verwendung einer Verbindung der Formel (I) oder eines physiologisch verträglichen Salzes derselben gemäß einem oder mehrerer der Ansprüche 1 bis 7 zur Herstellung eines Arzneimittel zur Behandlung und/oder Prophylaxe von koronarer Herzerkrankung, akutem Myokardinfarkt, instabiler Angina pectoris, Venenthrombose und venösen Thromboembolien, arteterieller Thrombose, Arteriosklerose, Insulinresistenz und makrovascularen Verletzungen in Typ-II Diabetis mellitus Patienten, Hypoxie, septischem Schock, Lungenentzündung und Lungenfibrose, sowie Krebserkrankungen, insbesondere Brustkrebs, Darmkrebs, Magenkrebs, Leberkrebs, Gehirntumoren, Eierstocktumoren, Speiseröhrenkrebs, Nierenkrebs, Muskeizellkarzinom.

**11.** Verwendung der Verbindung der Formel (I) oder eines physiologisch verträglichen Salzes derselben gemäß Anspruch 10 zur Gerinnungshemmung zur Behandlung von und/oder als Prophylaxe für thromboembolische Erkrankungen.

**12.** Arzneimittel mit einem Gehalt an mindestens einer Verbindung der Formel (I) gemäß einem oder mehrerer der Ansprüche 1 bis 7, wobei die Verbindung oder die Verbindungen der Formel (I) als solche in Substanz oder in Mischung mit einem oder mehrerem der üblichen pharmakologisch geeigneten Träger- oder Hilfsstoffen vorliegen.

**13.** Mikroorganismus Stachybotris atra ST002348, DSM 14952.

## Claims

**1.** A compound of the formula (I)

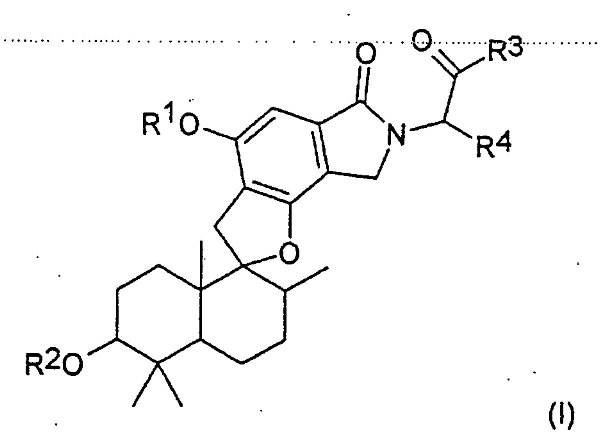

(I)

where

$R^1$ and $R^2$ independently of one another are H, $C_1$-$C_6$-alkyl, $C_2$-$C_6$-alkenyl, $C_2$-$C_6$-alkynyl or $C_5$-$C_{14}$-aryl, in which alkyl, alkenyl, alkynyl and aryl are unsubstituted or mono- to trisubstituted by a radical from the group consisting of -OH, =O, -O-$C_1$-$C_6$-alkyl, -O-$C_2$-$C_6$-alkenyl, -O-$C_5$-$C_{14}$-aryl, -NH-$C_1$-$C_6$-alkyl, -NH-$C_2$-$C_6$-alkenyl, -NH[-C(=O)-($C_1$-$C_6$-alkyl)], -NH[-C(=O)-($C_5$-$C_{14}$-aryl)], -$NH_2$ or halogen,

$R^3$ is -OH, -O-$R^1$ or -NH-$R^1$, and

$R^4$ is H, $C_1$-$C_6$-alkyl, $C_2$-$C_6$-alkenyl, $C_5$-$C_{14}$-aryl or -($C_1$-$C_6$-alkyl)-($C_5$-$C_{14}$-aryl),

or a physiologically tolerable salt of the compound of the formula (I).

**2.** A compound as claimed in claim 1, where $R^1$ and $R^2$ independently of one another are H or $C_1$-$C_6$-alkyl, preferably H.

**3.** A compound as claimed in one of claims 1 or 2, where $R^3$ is OH or -O-($C_1$-$C_6$-alkyl), preferably OH.

**4.** A compound as claimed in one of claims 1 to 3, where $R^4$ is $C_1$-$C_6$-alkyl or -($C_1$-$C_6$-alkyl)-($C_5$-$C_{14}$-aryl), preferably

benzyl, 2-butyl or 1-(2-methylpropyl).

5. A compound as claimed in one of claims 1 to 4, wherein the compound of the formula (I) has the following formula (II)

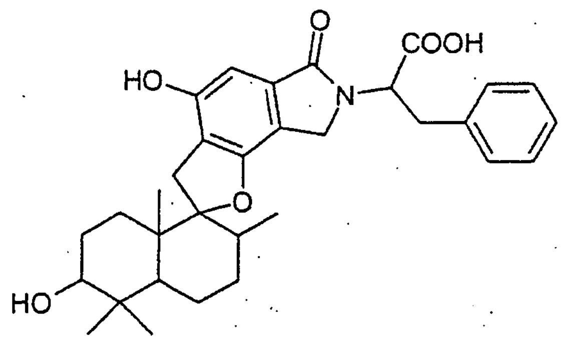

(II).

6. A compound as claimed in one of claims 1 to 4, wherein the compound of the formula (I) has the following formula (III)

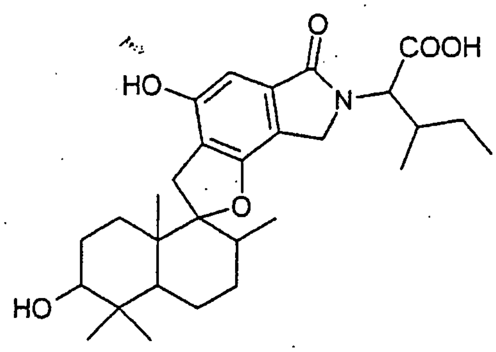

(III).

7. A compound as claimed in one of claims 1 to 4, wherein the compound of the formula (I) has the following formula (IV)

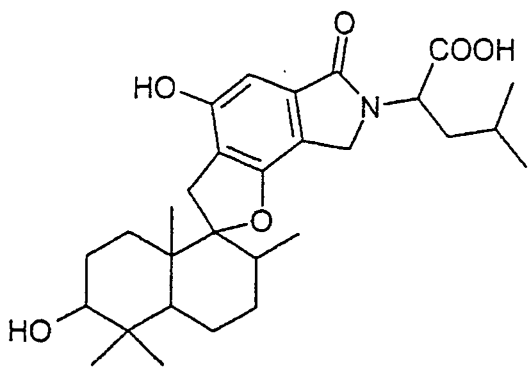

(IV).

8. A process for the preparation of a compound of the formula (I) as claimed in one of claims 1 to 7, which comprises fermenting the strain Stachybotris atra ST002348, DSM 14952, or one of its variants or mutants under suitable conditions in a culture medium until one or more of the compounds of the formula (I) accumulate in the culture medium and then isolating it from the culture medium and optionally converting it into a physiologically tolerable salt.

9. The use of a compound of the formula (I) or of a physiologically tolerable salt thereof as claimed in one or more of claims 1 to 7 for the production of a pharmaceutical.

10. The use of a compound of the formula (I) or of a physiologically tolerable salt thereof as claimed in one or more of claims 1 to 7 for the production of a pharmaceutical for the treatment and/or prophylaxis of coronary heart disease, acute myocardial infarct, unstable angina pectoris, venous thrombosis and venous thromboembolisms, arterial thrombosis, arteriosclerosis, insulin resistance and macrovascular injuries in type II diabetes mellitus patients, hypoxia, septic shock, pneumonia and pulmonary fibrosis, and cancers, in particular breast cancer, intestinal cancer, gastric cancer, hepatic cancer, brain tumors, ovarian tumors, esophageal cancer, renal cancer, muscle cell carcinoma.

11. The use of the compound of the formula (I) or of a physiologically tolerable salt thereof as claimed in claim 10 for the inhibition of clotting for the treatment of and/or as a prophylaxis for thromboembolic diseases.

12. A pharmaceutical containing at least one compound of the formula (I) as claimed in one or more of claims 1 to 7, the compound or the compounds of the formula (I) being present as such alone or as a mixture with one or more of the customary pharmacologically suitable vehicles or excipients.

13. The microorganism Stachybotris atra ST002348, DSM 14952.

**Revendications**

1. Composé de formule (I)

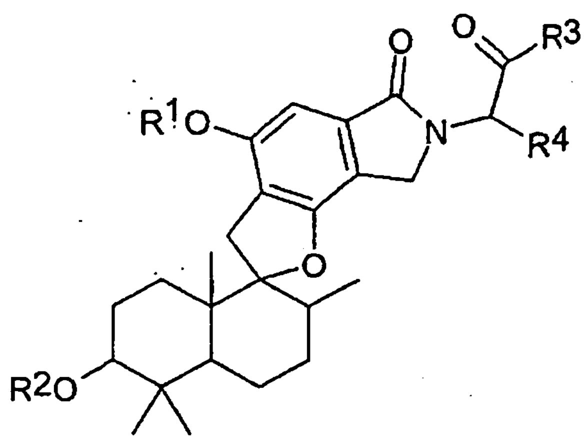

(I)

dans laquelle
$R^1$ et $R^2$ représentent chacun indépendamment de l'autre H ou un radical alkyle en $C_1$-$C_6$, alcényle en $C_2$-$C_6$, alcynyle en $C_2$-$C_6$ ou aryle en $C_5$-$C_{14}$, où les radicaux alkyle, alcényle, alcynyle et aryle sont non substitués, ou une à trois fois substitués par un résidu choisi dans le groupe consistant en les radicaux -OH, =O, -O-(alkyle en $C_1$-$C_6$), -O-(alcényle en $C_2$-$C_6$), -O-(aryle en $C_5$-$C_{14}$), -NH-(alkyle en $C_1$-$C_6$), -NH-(alcényle en $C_2$-$C_6$), -NH[C(=O)-(alkyle en $C_1$-$C_6$)], -NH[-C(=O)-(aryle en $C_5$-$C_{14}$)], -$NH_2$ ou halogéno,
$R^3$ est -OH, -O-$R^1$ ou -NH-$R^1$, et
$R^4$ est H ou un radical alkyle en $C_1$-$C_6$, alcényle en $C_2$-$C_6$, aryle en $C_5$-$C_{14}$, ou -(alkyle en $C_1$-$C_6$)-(aryle en $C_5$-$C_{14}$), ou un sel acceptables d'un point de vue physiologique du composé de formule (I).

2. Composé selon la revendication 1, dans lequel $R^1$ et $R^2$ représentent chacun indépendamment de l'autre H ou un radical alkyle en $C_1$-$C_6$, de préférence H.

3. Composé selon l'une des revendications 1 ou 2, dans lequel $R^3$ est OH ou un radical -O-(alkyle en $C_1$-$C_6$), de préférence OH.

4. Composé selon l'une des revendications 1 à 3, dans lequel $R^4$ est un radical alkyle en $C_1$-$C_6$ ou - (alkyle en $C_1$-$C_6$)-(aryle en $C_5$-$C_{14}$), de préférence un groupe benzyle, 2-butyle ou 1-(2-méthyl-propyle).

5. Composé selon l'une des revendications 1 à 4, **caractérisé en ce que** le composé de formule (I) a la formule (II) ci-après :

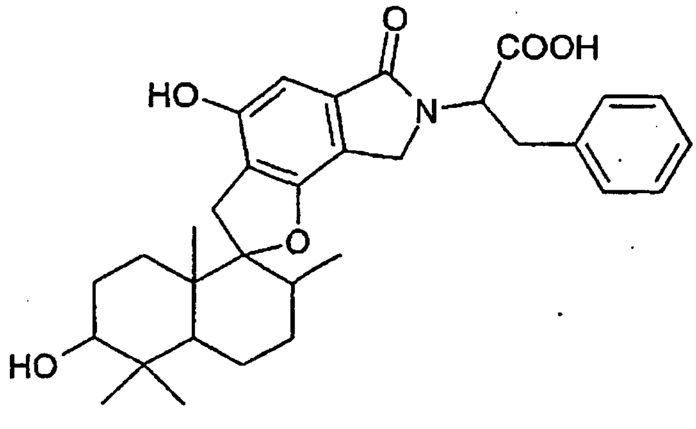

(II).

6. Composé selon l'une des revendications 1 à 4, **caractérisé en ce que** le composé de formule (I) a la formule (III) ci-après :

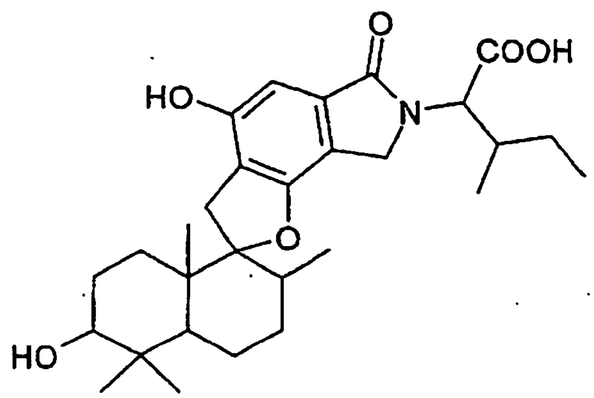

(III).

7. Composé selon l'une des revendications 1 à 4, **caractérisé en ce que** le composé de formule (I) a la formule (IV) ci-après :

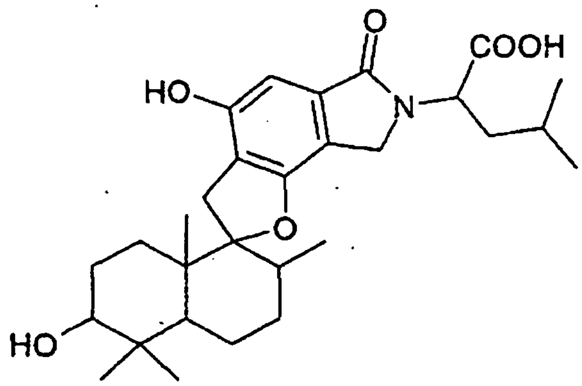

(IV).

8. Procédé de préparation d'un composé de formule (I) selon l'une des revendications 1 à 7, **caractérisé en ce qu'**on fermente dans un milieu de culture la souche Stachybotris atra ST002348, DSM 14952, ou l'un de ses variants ou mutants dans des conditions appropriées, jusqu'à ce qu'un ou plusieurs des composés de formule (I) s'accumulent dans le milieu de culture, puis on les isole du milieu de culture, et éventuellement on les convertit en un sel acceptable d'un point de vue physiologique.

9. Utilisation d'un composé de formule (I) ou d'un sel acceptable d'un point de vue physiologique de ce dernier selon l'une ou plusieurs des revendications 1 à 7 pour préparer un médicament.

10. Utilisation d'un composé de formule (I) ou d'un sel acceptable d'un point de vue physiologique de ce dernier selon l'une ou plusieurs des revendications 1 à 7 pour préparer un médicament destiné au traitement et/ou à la prophylaxie de la maladie coronarienne, de l'infarctus du myocarde aigu, de l'angine de poitrine instable, de la thrombose veineuse et des thromboembolies veineuses, de la thrombose artérielle, de l'artériosclérose, de l'insulino-résistance et des lésions macrovasculaires chez les patients présentant un diabète sucré de type II, de l'hypoxie, du choc septique, des inflammations pulmonaires et de la fibrose pulmonaire, ainsi que des maladies cancéreuses, en particulier le cancer du sein, le cancer de l'intestin, le cancer de l'estomac, le cancer du foie, les tumeurs cérébrales, les tumeurs des ovaires, le cancer de l'oesophage, le cancer des reins, le cancer des cellules musculaires.

11. Utilisation du composé de formule (I) ou d'un sel acceptable d'un point de vue physiologique de ce dernier selon la revendication 10, pour assurer une inhibition de la coagulation pour le traitement et/ou la prophylaxie de maladies thrombo-emboliques.

12. Médicament contenant au moins un composé de formule (I) selon l'une ou plusieurs des revendications 1 à 7, pour lequel le composé ou les composés de formule (I) sont utilisés en l'état sans additif, ou en mélange avec un ou plusieurs des excipients ou adjuvants usuels, acceptables d'un point de vue pharmacologique.

13. Le microorganisme Stachybotris atra ST002348, DSM 14952.